(19) Europäisches Patentamt
European Patent Office
Office européen des brevets

(11) **EP 3 991 716 A1**

(12) **EUROPEAN PATENT APPLICATION**
published in accordance with Art. 153(4) EPC

(43) Date of publication:
**04.05.2022 Bulletin 2022/18**

(21) Application number: **19935112.3**

(22) Date of filing: **20.12.2019**

(51) International Patent Classification (IPC):
**A61K 8/73** (2006.01)  **A61Q 19/08** (2006.01)
**A61Q 19/00** (2006.01)

(86) International application number:
**PCT/CN2019/127012**

(87) International publication number:
**WO 2020/258775 (30.12.2020 Gazette 2020/53)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.06.2019 CN 201910559008**

(71) Applicant: **Bioregen Biomedical (Changzhou) Co., Ltd.**
**Changzhou, Jiangsu 213125 (CN)**

(72) Inventors:
• **WANG, Yunyun**
**hangzhou, Jiangsu 213125 (CN)**

• **WANG, Xinyu**
**hangzhou, Jiangsu 213125 (CN)**
• **SONG, Wenjun**
**hangzhou, Jiangsu 213125 (CN)**
• **ZHANG, Hongchen**
**hangzhou, Jiangsu 213125 (CN)**
• **WANG, Kun**
**hangzhou, Jiangsu 213125 (CN)**
• **SHU, Xiaozheng**
**hangzhou, Jiangsu 213125 (CN)**

(74) Representative: **Hynell Intellectual Property AB**
**Kabyssgatan 4D**
**120 30 Stockholm (SE)**

(54) **HYALURONIC ACID SKIN PROTECTION COMPOSITION, AND PREPARATION METHOD THEREFOR AND APPLICATION THEREOF**

(57)    The present disclosure discloses a hyaluronic acid skin care composition, including: 1) a thiolated hyaluronic acid derivative; and 2) a hyaluronic acid or salt of at least one molecular weight of thereof. The composition has unique advantages when used for skin care, not only has multiple skin protection effects of the hyaluronic acid itself, but also has strong anti-oxidation ability, and can effectively scavenge reactive oxygen species.

FIG. 3

## Description

### TECHNICAL FIELD

The present disclosure relates to the field of biomedicine, particularly relates to a hyaluronic acid skin care composition, and also relates to a preparation method and application of the hyaluronic acid skin care composition.

### BACKGROUND

During normal metabolism of human body, there is a dynamic balance between an oxidation process and a reduction process. However, under the action of a variety of endogenous factors (such as chronic or acute infections) and exogenous factors (such as environmental pollution and ultraviolet radiation), the oxidation reaction may be dominant, and oxidative stress may occur, resulting in a variety of harmful reactive oxygen species. These reactive oxygen species have strong oxidation capacity, and may attack lipids, proteins and DNA to damage tissues and cells of the body, thus causing various chronic diseases and an aging effect (Gutteridge et al., British Medical Bulletin 1999, 55:49-57).

Skin is the largest organ in the human body. Studies have shown that up to 80% of skin aging is related to reactive oxygen species. Due to the destructive effect of reactive oxygen species, cells and extracellular matrix that maintain the balance of the skin are destroyed and reduced, and its immune repair ability is rapidly reduced, finally resulting in various skin aging and pathological symptoms, such as dryness, roughness, dullness, looseness, and wrinkles.

Moisturizing is an important part of skin care, and can effectively improve some symptoms of skin aging, such as dullness, fine wrinkles, roughness, scaling and itching. The hydration level of the skin is closely related to the amount of hyaluronic acid. With the increase of age and the destruction of reactive oxygen species, the amount of hyaluronic acid in the skin decreases, which weakens the hydration function of the skin to cause aging (Agren et al., Free Radical Biol. Med. 1997, 23: 996-1001; Stern et al., Clinics in Dermatology 2008, 26: 106-122). Hyaluronic acid is an acidic mucopolysaccharide inherent in the human body. It has been widely used in various skin care products, is a super moisturizing agent naturally present in human skin, and can hold moisture 1,000 times its own weight. The moisturizing effect is the most important effect of hyaluronic acid in skin care products. Topical use of hyaluronic acid for moisturizing is an important measure to delay skin aging symptoms and partly restore youthfulness. An aqueous solution of hyaluronic acid has relatively strong viscoelasticity and lubricity. It can be applied to the surface of skin to form a moisturizing breathable film to keep the skin moist and bright. Hyaluronic acid has unique moisturizing property, which is hardly impacted by the relative humidity of a surrounding environment. Even in extreme cases where the relative humidity is very low, it can still capture moisture from air and supply the moisture to the skin. On the contrary, under extremely dry conditions, traditional moisturizers (such as sorbitol and polyethylene glycol) absorb moisture from the skin, which may aggravate skin dehydration symptoms (Smejkalova, et al., Harry's Cosmeticology 9th Edition Vol.2. Part 4.1.3, pp.605-622; Publisher: Chemical Publishing Company, 2015).

The moisturizing effect of hyaluronic acid is positively correlated with its molecular weight, and the high molecular weight hyaluronic acid has better moisturizing, film-forming and lubricating properties. The high molecular weight hyaluronic acid added in current skin care products is usually greater than 800 KDa (800,000 Daltons). When applied to the surface of skin, it not only has a good moisturizing effect, but also can achieve a lifting and firming effect, effectively improve rough skin and make the skin more delicate and smoother (Biomatrix Inc. Hyaluronan Vol.2: Biomedical, Medical and Clinical Aspects, pp.285-288; Publisher: Woodhead Publishing Limited, 2002).

The hyaluronic acid has a very wide molecular weight distribution, and different molecular weights have different physical, chemical and biological properties. Studies have shown that low-medium molecular weight hyaluronic acids with molecular weights of less than 500 KDa can penetrate superficial stratum corneum of the skin in a small amount, hydrate and soften the stratum corneum, and establish a bridge with the endogenous hyaluronic acid in the deep epidermis, which is beneficial for absorption of nutrients and excretion of metabolic waste. The transdermal absorption property of hyaluronic acid is negatively correlated with a molecular weight, and the decrease in molecular weight is helpful for transdermal absorption. Studies have also shown that small molecular hyaluronic acids (oligosaccharides with 6 to 20 disaccharide repeating units, that is, with a molecular weight of 2.4 KDa to 8 KDa) can also slightly improve blood flow (promote micro-angiogenesis), and promote the growth of fibroblasts and the secretion and synthesis of endogenous macromolecular hyaluronic acids. Therefore, skin care products containing low molecular weight hyaluronic acids can repair and nourish basal skin to a certain extent (Farwick et al., SOFW-Journal 2008, 134: 1-6; Smejkalova et al., Harry's Cosmeticology 9th Edition Vol.2. Part 4.1.3, pp.605-622; Publisher: Chemical Publishing Company, 2015).

Therefore, existing hyaluronic acid skin care products usually contain a plurality of hyaluronic acids with different molecular weights to achieve an integrative skin protection effect. CN 106109265B discloses a hyaluronic acid moisturizing composition, including 60-62wt% of hyaluronic acid with a molecular weight of 1,100 KDa or a salt thereof, 18-20wt% of hyaluronic acid with a molecular weight of 400 KDa or a salt thereof, and 18-20% of hydrolyzed hyaluronic acid with a molecular weight of 6 KDa or a salt thereof. CN 106137786B discloses a hyaluronic acid anti-aging composition, including 60-62wt% of hydrolyzed hyaluronic acid with a molecular weight of 7 KDa or a salt thereof, 18-20wt% of hyaluronic acid with a molecular weight of 320 KDa or a salt thereof, and 18-20wt% of hyaluronic acid with a molecular weight of 1,100 KDa or a salt thereof. CN 101450028A discloses an efficient moisturizing hyaluronic acid stock solution, which is composed of high molecular weight sodium hyaluronate with an average molecular weight greater than 2,600 KDa and low molecular weight sodium hyaluronate with a molecular weight less than 10 KDa. CN 101500535A discloses a composition with several hyaluronic acid fractions for cosmetic and medical purposes, including at least two hyaluronic acid fractions or salts thereof, one of which has an average molecular weight of 8 KDa to 100 KDa, and the other fraction has an average molecular weight of 100 KDa to 500 KDa.

In addition, the hyaluronic acid can also quench reactive oxygen species with the destroying and decomposing of itself at the same time, and has a weak anti-inflammatory effect. However, this ability to quench reactive oxygen species is very weak and is not sufficient to scavenge the reactive oxygen species effectively (Yamazaki et al., Pathophysiology 2003, 9:215-220.; Stern et al., Biotechnology Advances 2007, 25:537-557; Serban et al., Biomaterials 2008, 29: 1388-1399; CN 106137786B).

Therefore, although the current hyaluronic acid skin care products can improve some symptoms of skin aging to a certain extent, they cannot effectively scavenge reactive oxygen species and fail to effectively eliminate root causes of skin aging.

## BRIEF SUMMARY

In order to solve the problem that hyaluronic acids cannot effectively scavenge reactive oxygen species when used for skin care in the prior art, the present application provides a new hyaluronic acid skin care composition, which not only has moisturizing and other skin care functions of a hyaluronic acid, but also has enhanced anti-oxidation capacity, can effectively scavenge reactive oxygen species, and therefore, can more effectively improve skin aging symptoms and better solve skin aging problems.

The present disclosure is realized through the following technical solution:

A hyaluronic acid skin care composition, including:

1) a thiolated hyaluronic acid derivative; and

2) a hyaluronic acid or salt of at least one molecular weight thereof.

The hyaluronic acid or salt thereof in the present disclosure includes a sodium salt, a potassium salt, a zinc salt, a calcium salt, etc., preferably a sodium salt and a potassium salt, and particularly preferably a sodium salt.

In the present disclosure, the thiolated hyaluronic acid derivative used contains thiol groups, which can effectively inhibit oxidative stress directly caused by reactive oxygen species, and can prevent lipid, protein and DNA damage; in addition, it can also be complexed with divalent iron ions to indirectly inhibit the formation of hydroxyl radicals and the like.

The thiolated hyaluronic acid derivative in the present disclosure refers to a hyaluronic acid derivative containing thiol groups, which can be prepared by the thiolation of the hyaluronic acid or salt thereof, and also includes the thiolated derivatives prepared by a further thiolation of various hyaluronic acid derivatives. The side-chain carboxyl group, the side-chain hydroxyl group, the reducing end group and the like of a hyaluronic acid or salt or derivative thereof are usually reactive functional groups that can undergo thiolation. WO 2009/006780A1 discloses a method for preparing a plurality of thiolated hyaluronic acid derivatives.

The thiolation of the side-chain carboxyl group of the hyaluronic acid can be realized through a chemical method of amino (hydrazide group)/carbodiimide coupling, the carbodiimide including 1-ethyl-3-(3-dimethylaminopropyl) carbodiimide hydrochloride, etc. Generally, the side-chain carboxyl group is activated by the carbodiimide to form an intermediate product, that is, a diamine or dihydrazide containing a disulfide bond is nucleophilically substituted to generate an intermediate product, and finally the disulfide bonds are reduced into thiol groups to obtain the thiolate hyaluronic acid derivative (Shu et al., Biomacromolecules 2002, 3: 1304-1311; Aeschlimann et al., US 7,196,180B1). A primary amine containing a thiol (or thiol protecting) group can also be used instead of the diamine or dihydrazide containing a disulfide bond to prepare (or remove the thiol protecting group from the resulting intermediate product) a thiolated hyaluronic acid derivative (Gianolio et al., Bioconjugate Chemistry 2005, 16:1512-1518; Kafedjiiski et al., International Journal of Pharmaceutics 2007, 343:48-58). Another method of thiolation of the side-chain carboxyl group is to directly react with a carbodiimide containing a disulfide bond (such as 2, 2'-dithiobis (N-ethyl (N'-ethyl carbodiimide)))(Bulpitt et al., US 6,884,788).

The thiolation of the side-chain hydroxyl group of the hyaluronic acid can be realized through a nucleophilic ring-opening reaction of the hydroxyl group and ethylene sulfide under an alkaline condition (Serban et al., Biomaterials 2008, 29: 1388-1399). The thiolation of the side-chain hydroxyl group of the hyaluronic acid can also be carried out indirectly. For example, the hydroxyl group is first carboxymethylated under an alkaline condition, and then the thiolation of the carboxyl group is carried out (Prestwich et al., WO 2005/056608A1).

The reducing end group of the hyaluronic acid is also an active group that can undergo thiolation, and the terminal thiol group can be introduced chemically (Inoue et al., Carbohydrate Research 1985, 141:99-110). In addition, the hyaluronic acid derivative, such as acetylated sodium hyaluronate disclosed in CN 109206537A, can also be modified by the above-mentioned thiolation method to obtain the thiolated hyaluronic acid derivative of the present disclosure.

In the present disclosure, the thiol content in the thiolated hyaluronic acid derivative can be determined by Ellman's reagent method or $^1$H NMR absorption spectrogram (Shu et al., Biomacromolecules 2002, 3: 1304-1311), and is expressed as a number of micromoles of thiol groups per gram of the thiolated hyaluronic acid derivative ($\mu$mol/g). The thiol substitution degree of the thiolated hyaluronic acid derivative is usually defined as a percentage of a number of thiol groups introduced per 100 hyaluronic acid disaccharide repeating units. Since the molecular weight of the disaccharide repeating units of the thiolated hyaluronic acid derivative is approximately equal to 400 Da, it can be calculated from the thiol content and the thiol substitution degree. For example, when the thiol content is 125 $\mu$mol/g, 5 thiol groups are introduced per 100 disaccharide units, that is, the thiol substitution degree is 5%.

In the present disclosure, various thiol substitution degrees of thiolated hyaluronic acid derivatives can be used to prepare the hyaluronic acid skin care composition of the present disclosure, such as the thiolated hyaluronic acid derivative with a thiol substitution degree (thiol content) of 26.8% to 66.8% (670 $\mu$mol/g to 1670 $\mu$mol/g) reported by Shu et al. (Shu et al., Biomacromolecules 2002, 3:1304-1311), the thiolated derivative with a thiol substitution degree (thiol content) of 4% to 14% (100 $\mu$mol/g to 350 $\mu$mol/g) reported by Serban et al. (Serban et al., Biomaterials 2008, 29: 1388-1399), the thiolated hyaluronic acid derivative with a thiol substitution degree (thiol content) $\leq$ 4.5% ($\leq$ 112.5 $\mu$mol/g) reported by Shu et al. (US 2013/0338099A1), and a plurality of thiolated hyaluronic acid derivatives with different thiol content disclosed in CN101622017B.

In the present disclosure, the thiol content is a decisive factor for the anti-oxidation ability of the thiolated hyaluronic acid derivative. Compared with an unmodified hyaluronic acid, the thiolated hyaluronic acid derivative used in the present disclosure has significantly enhanced anti-oxidation ability, and its scavenging ability towards free radical can be enhanced by 10 to 40 times, or even higher. On the one hand, the increase in the thiol content helps to enhance the scavenging ability towards reactive oxygen species; but on the other hand, when the thiol content is relatively low, it may help to better maintain the original structure of a hyaluronic acid and the skin care function of the hyaluronic acid itself. Therefore, the thiolated hyaluronic acid derivative used in the present disclosure combines the above two factors. Its thiol content is usually less than 500 $\mu$mol/g, preferably less than 100 $\mu$mol/g, and particularly preferably between 20 $\mu$mol/g and 50 $\mu$mol/g.

In the present disclosure, the thiol content determines the anti-oxidation ability of the thiolated hyaluronic acid derivative to a great extent, and the molecular weight has a little impact on its anti-oxidation ability. In the present disclosure, the thiolated hyaluronic acid derivative used usually has a molecular weight of between 1 KDa and 10,000 KDa, preferably between 1.6 KDa and 3,000 KDa, and particularly preferably between 2.4 KDa and 1,500 KDa.

The molecular weight in the present disclosure refers to an average molecular weight.

In the hyaluronic acid skin care composition of the present disclosure, the thiolated hyaluronic acid derivative usually has a concentration of less than 5 mg/ml, preferably less than 2 mg/ml, and particularly preferably less than 1 mg/ml.

The hyaluronic acid skin care composition of the present disclosure may also include two or more thiolated hyaluronic acid derivatives, and these thiolated derivatives may have different molecular weights and/or thiol contents.

The hyaluronic acid skin care composition of the present disclosure also includes a hyaluronic acid or salt of at least one molecular weight thereof, and the molecular weight is usually between 1 KDa and 10,000 KDa, preferably between 1.6 KDa and 3,000 KDa, and particularly preferably between 2.4 KDa and 2,800 KDa.

In the hyaluronic acid skin care composition of the present disclosure, when hyaluronic acids or salts of two or more molecular weights thereof are included, preferably at least one of the hyaluronic acids or salts thereof has a molecular weight of between 1.6 KDa and 500 KDa and at least one of the hyaluronic acids or salts thereof has a molecular weight of between 800 KDa and 3,000 KDa, and particularly preferably at least one of the hyaluronic acids or salts thereof has a molecular weight of between 2.4 KDa and 8 KDa and at least one of the hyaluronic acids or salts thereof has a molecular weight of between 1,000 KDa and 2,800 KDa.

In the hyaluronic acid skin care composition of the present disclosure, the hyaluronic acid or salt thereof included usually has a concentration of less than 10 mg/ml, preferably less than 8 mg/ml, and particularly preferably less than 4 mg/ml.

In the present disclosure, vitamins, amino acids, traditional moisturizers (such as sorbitol and polyethylene glycol), antioxidants, etc. can also be added to the hyaluronic acid skin care composition.

The hyaluronic acid skin care composition of the present disclosure can usually be terminally sterilized (such as moist heat sterilization) in a manner well known to those skilled in the art, and preservatives may not be necessary, which can effectively eliminate the adverse reactions caused by the preservatives. On the other hand, if necessary, preservatives/bacteria inhibitors well known to those skilled in the art can also be appropriately added to the hyaluronic acid care protection composition of the present disclosure.

Compared with the hyaluronic acid anti-aging composition disclosed in CN106137786B, the composition of the present disclosure has more significant scavenging ability towards free radical, and the scavenging rate can be increased by 100% to 1000% or more. Therefore, the composition of the present disclosure has unique anti-aging advantages when used for skin care.

Another objective of the present disclosure is to provide a preparation method of the hyaluronic acid skin care composition. The preparation method of the hyaluronic acid skin care composition is characterized in that various components are uniformly mixed according to a set ratio. In order to better maintain the anti-oxidation ability of the thiolated hyaluronic acid derivative included in the composition of the present disclosure, the components can also be filled in a sealed container under the protection of an inert atmosphere after uniform mixing.

Another objective of the present disclosure is to provide an application of the hyaluronic acid skin care composition. The application of the hyaluronic acid skin care composition refers to an application in skin cleansing, care, and cosmetics, and has moisturizing and anti-oxidation effects.

The hyaluronic acid skin care composition not only has the moisturizing skin care effect of the hyaluronic acid itself, but also has strong anti-oxidation effect, can effectively scavenge reactive oxygen species, and can thus effectively improve the symptoms of skin aging and better solve the problems of skin aging; and it can be used in all forms of cosmetics and cleansing products, including toners, essences, gels, lotions, creams, masks, makeup, soaps, facial cleansers and shower gels.

The present disclosure has the following advantages:

The composition of the present disclosure includes a thiolated hyaluronic acid derivative, which can effectively quench reactive oxygen species, inhibit oxidative stress directly caused by them, prevent lipid, protein and DNA damage, and can also indirectly inhibit the formation of reactive oxygen species such as hydroxyl radicals. In addition, the composition of the present disclosure further includes one molecular weight or more different molecular weights of hyaluronic acids or salts thereof, which can form a combination of different molecular weights to fully exert multiple skin protection effects of moisturizing, softening stratum corneum, repairing and nourishing basal skin, etc. of different molecular weights of hyaluronic acids.

The composition of the present disclosure has unique advantages when used for skin care. It not only has multiple skin care effects of the hyaluronic acid itself, but also has strong anti-oxidation ability, can effectively scavenge reactive oxygen species, and can thus have good skin care effects of effectively improving skin aging symptoms, such as increasing skin elasticity and reducing wrinkles.

## BRIEF DESCRIPTION OF THE DRAWINGS

FIG. 1 shows scavenging rates of DPPH free radicals by thiolated hyaluronic acid derivatives with different concentrations and thiol contents.

FIG. 2 shows scavenging rates of DPPH free radicals by thiolated hyaluronic acid derivatives with different molecular weights.

FIG. 3 shows scavenging rates of DPPH free radicals by hyaluronic acid skin care compositions (including a molecular weight of sodium hyaluronate).

FIG. 4 shows scavenging rates of DPPH free radicals by hyaluronic acid skin care compositions (including two molecular weights of sodium hyaluronate).

FIG. 5 shows moisturizing properties of the hyaluronic acid skin care compositions.

FIG. 6 shows effects of the hyaluronic acid skin care compositions on skin elasticity.

FIG. 7 shows effects of the hyaluronic acid care protection compositions on depths of skin wrinkles.

## DETAILED DESCRIPTION OF THE DISCLOSURE

The implementation scheme of the present disclosure will be described in detail below in conjunction with embodiments, but a person skilled in the art would understand that the following embodiments are only used to illustrate the present disclosure and should not be regarded as limiting the scope of the present disclosure.

Embodiment 1: anti-oxidation effects of thiolated hyaluronic acid derivatives with different thiol content in scavenging free radicals

Free radical scavenging performance of thiolated hyaluronic acid derivatives used in the present disclosure was evaluated by a 1,1-diphenyl-2-trinitrophenylhydrazine (DPPH) test method. DPPH was widely used in quantitative determination of anti-oxidation abilities of biochemical substances. This method was based on the characteristics that DPPH free radicals had single electrons and had a strong absorption at a wavelength of 517 nm, and its alcohol solution was purple. In the presence of a free radical scavenger, its absorption gradually disappeared due to pairing with the single electrons, and the degree of fading was quantitatively related to the number of electrons it accepts. Therefore, a spectrophotometer can be used for rapid quantitative analysis (Sharma et al., Food Chemistry 2009, 113: 1202-1205).

Thiol groups of hyaluronic acids were prepared by the method reported by Shu et al. (Shu et al., Biomacromolecules 2002, 3:1304-1311), and their thiol contents were 18 $\mu$mol/g, 38 $\mu$mol/g, 57 $\mu$mol/g, 108 $\mu$mol/g, 266 $\mu$mol/g, 416 $\mu$mol/g, and 530 $\mu$mol/g respectively.

Sodium hyaluronate raw materials and the thiolated sodium hyaluronate derivatives were respectively dissolved in a phosphate buffer solvent (pH 7.4) to obtain test samples with a final concentration of 0.1 mg/mL, 0.5 mg/mL or 1 mg/mL.

0.1 mmol/L DPPH solution was prepared from absolute ethanol and stored in the dark.

2 mL of test sample solution and 2 mL of DPPH solution were added into the same test tube, the two solutions were shaken well and stood in the dark at room temperature for 30 min, and then the absorbance (A1) at 517 nm was measured; meanwhile, 2 mL of DPPH solution and 2 mL of phosphate buffer solvent were mixed and then the absorbance (A0) at 517 nm was measured, and 2 mL of test sample solution and 2 mL of absolute ethanol were mixed and then the absorbance (A2) at 517 nm was measured.

The free radical scavenging ability was calculated as follows:

$$\text{Scavenging rate} = \left(1 - \frac{A_1 - A_2}{A_0}\right) \times 100\%$$

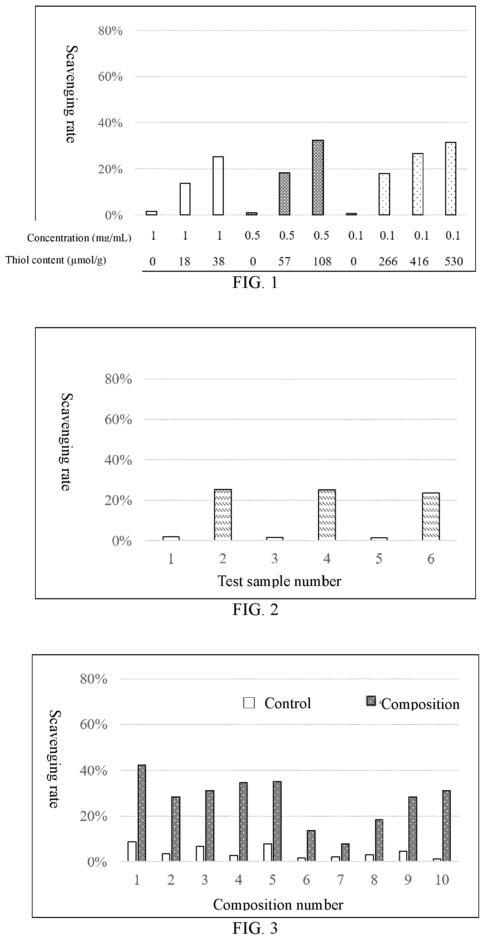

See FIG. 1 for the results of this embodiment. The test results showed that the scavenging rate of DPPH free radicals was positively correlated with the number of thiol groups in the test sample, and the scavenging rate by the thiolated sodium hyaluronate derivative was about 10 to 40 times higher than that by the sodium hyaluronate raw material.

Embodiment 2: anti-oxidation effects of thiolated hyaluronic acid derivatives with different molecular weights in scavenging free radicals

Thiolated hyaluronic acid derivatives were prepared from 180 KDa, 300 KDa and 1,500 KDa sodium hyaluronate as raw materials by using the method reported by Shu et al. (Shu et al., Biomacromolecules 2002, 3:1304-1311), and their thiol contents were 38 $\mu$mol/g, 33 $\mu$mol/g and 40 $\mu$mol/g respectively.

The sodium hyaluronate raw materials and the thiolated sodium hyaluronate derivatives were respectively dissolved in a phosphate buffer solvent (pH 7.4) to obtain test sample solutions described in the following table.

| Test sample number | Composition | | Test sample solution Thiol content |
| --- | --- | --- | --- |
| | Sodium hyaluronate | Thiolated sodium hyaluronate | |
| 1 | 1 mg/ml (180 KDa) | 0 | 0 |
| 2 | 0 | 1 mg/ml (180 KDa) | $38 \times 10^{-3}$ $\mu$mol/mL |
| 3 | 1.15 mg/ml (300 KDa) | 0 | 0 |
| 4 | 0 | 1.15 mg/ml (300 KDa) | $38 \times 10^{-3}$ $\mu$mol/mL |
| 5 | 0.95 mg/ml (1,500 KDa) | 0 | 0 |
| 6 | 0 | 0.95 mg/ml (1,500 KDa) | $38 \times 10^{-3}$ $\mu$mol/mL |

The anti-oxidation effects of the above sample solutions in scavenging free radicals were measured according to the DPPH method described in Embodiment 1.

See FIG. 2 for the results of this embodiment. Test samples 1, 3 and 5 were sodium hyaluronate control solutions with different molecular weights, which had low DPPH free radical scavenging rate. Test samples 2, 4 and 6 were test sample solutions of thiolated hyaluronic acid derivatives with different molecular weights, which had the same thiol content ($38 \times 10^{-3}$ $\mu$mol/mL) and similar DPPH free radical scavenging rate of about 15 times higher than the control solutions.

Embodiment 3: preparation of hyaluronic acid skin care compositions (including a molecular weight of sodium hyaluronate)

Thiolated hyaluronic acid derivatives used were prepared from 180 KDa sodium hyaluronate as a raw material by using the method reported by Shu et al. (Shu et al., Biomacromolecules 2002, 3:1304-1311), and their thiol contents were 18 μmol/g, 38 μmol/g, 57 μmol/g, 108 μmol/g, 266 μmol/g, 416 μmol/g, and 530 μmol/g respectively.

The sodium hyaluronate had molecular weights of 1.6 KDa, 3 KDa, 8 KDa, 50 KDa, 180 KDa, 500 KDa, 800 KDa, 1,500 KDa, and 2,700 KDa.

The thiolated hyaluronic acid derivative of any thiol content above was dissolved in distilled water to obtain a 6 mg/mL solution; the sodium hyaluronate of any molecular weight above was dissolved in distilled water to obtain a 12 mg/mL solution; and then the two were mixed uniformly at a ratio of 0.9:0.1 to 0.1:0.9 to obtain hyaluronic acid skin care compositions of different proportions. In these compositions, the thiolated hyaluronic acid derivatives had any concentration of between 5.4 mg/mL and 0.6 mg/mL, and the sodium hyaluronate had any concentration of between 1.2 mg/mL and 10.8 mg/mL.

In the compositions obtained, distilled water was added at a volume ratio of 1:0.08 to 0.08:1, and diluted compositions were obtained after uniform mixing. In the diluted compositions, the thiolated hyaluronic acid derivatives had any concentration of between 5.0 mg/mL and 0.04 mg/mL, and the sodium hyaluronate had any concentration of between 0.09 mg/mL and 10.0 mg/mL.

The above compositions were filled in glass ampoules under the protection of an inert atmosphere (nitrogen) and sealed for later use.

Embodiment 4: effects of hyaluronic acid skin care compositions (including a molecular weight of sodium hyaluronate) in scavenging free radicals

Ten hyaluronic acid care compositions (compositions of which were shown in the following table) prepared in Embodiment 3 were selected, and their anti-oxidation effects in scavenging free radicals were measured according to the method described in Embodiment 1.

| Composition number | | 1 | 2 | 3 | 4 | 5 | 6 | 7 | 8 | 9 | 10 |
|---|---|---|---|---|---|---|---|---|---|---|---|
| Thiolated hyaluronic acid derivative | Thiol content (μmol/g) | 18 | 18 | 38 | 38 | 108 | 108 | 108 | 266 | 416 | 530 |
| | Concentration (mg/mL) | 5 | 2 | 1 | 2 | 0.5 | 0.2 | 0.1 | 0.1 | 0.1 | 0.1 |
| Sodium hyaluronate | Molecular weight (KDa) | 1.6 | 180 | 3 | 500 | 8 | 1,500 | 800 | 500 | 50 | 2,700 |
| | Concentration (mg/mL) | 10 | 4 | 8 | 4 | 10 | 1 | 2 | 3 | 8 | 1 |

The thiolated hyaluronic acid derivative in each composition was substituted by a sodium hyaluronate raw material of the same molecular weight as a control.

See FIG. 3 for the results of this embodiment. The compositions of various proportions all had a significant anti-oxidation ability of scavenging free radicals, which was 3.4 to 26 times stronger than that of the control.

Embodiment 5: preparation of hyaluronic acid skin care compositions (including two molecular weights of sodium hyaluronate)

Thiolated hyaluronic acid derivatives used were prepared from 180 KDa sodium hyaluronate as a raw material by using the method reported by Shu et al. (Shu et al., Biomacromolecules 2002, 3:1304-1311), and their thiol contents were 18 μmol/g, 38 μmol/g, 57 μmol/g, 108 μmol/g, 266 μmol/g, 416 μmol/g, and 530 μmol/g respectively.

The sodium hyaluronate had molecular weights of 1.6 KDa, 3 KDa, 8 KDa, 50 KDa, 180 KDa, 500 KDa, 800 KDa, 1,500 KDa, and 2,700 KDa.

The thiolated hyaluronic acid derivative of any thiol content above was dissolved in distilled water to obtain a 6 mg/mL solution; the sodium hyaluronate (at any weight ratio) of any molecular weight above was dissolved in distilled water to obtain a solution with a total concentration of 12 mg/mL; and then the two were mixed uniformly at a ratio of 0.9:0.1 to 0.1:0.9 to obtain hyaluronic acid skin care compositions of different proportions. In these compositions, the thiolated hyaluronic acid derivatives had any concentration of between 5.4 mg/mL and 0.6 mg/mL, and the sodium hyaluronate had any total concentration of between 1.2 mg/mL and 10.8 mg/mL.

In the compositions obtained, distilled water was added at a volume ratio of 1:0.08 to 0.08:1, and diluted compositions were obtained after uniform mixing. In the diluted compositions, the thiolated hyaluronic acid derivatives had any concentration of between 5.0 mg/mL and 0.04 mg/mL, and the total sodium hyaluronate had any concentration of between 0.09 mg/mL and 10.0 mg/mL.

The above compositions were filled in glass ampoules under the protection of an inert atmosphere (helium) and sealed for later use.

Embodiment 6: effects of hyaluronic acid skin care compositions (including two molecular weights of sodium hyaluronate) in scavenging free radicals

Ten hyaluronic acid protection compositions (compositions of which were shown in the following table) prepared in Embodiment 5 were selected, and their anti-oxidation effects in scavenging free radicals were measured according to the method described in Embodiment 1.

| Composition number | | 1 | 2 | 3 | 4 | 5 | 6 | 7 | 8 | 9 | 10 |
|---|---|---|---|---|---|---|---|---|---|---|---|
| Thiolated hyaluronic acid derivative | Thiol content ($\mu$mol/g) | 38 | 38 | 38 | 38 | 38 | 57 | 57 | 57 | 57 | 57 |
| | Concentration (mg/mL) | 2 | 2 | 1 | 1 | 1 | 1 | 1 | 0.5 | 0.5 | 0.5 |
| Sodium hyaluronate 1 | Molecular weight (KDa) | 800 | 1500 | 1500 | 800 | 2700 | 800 | 1500 | 1500 | 800 | 2700 |
| | Concentration (mg/mL) | 2 | 1 | 1 | 1 | 0.5 | 1 | 0.5 | 1 | 2 | 0.5 |
| Sodium hyaluronate 2 | Molecular weight (KDa) | 500 | 180 | 50 | 8 | 3 | 180 | 1.6 | 50 | 8 | 3 |
| | Concentration (mg/mL) | 2 | 3 | 7 | 9 | 10 | 2 | 5 | 4 | 3 | 3 |

The thiolated hyaluronic acid derivative in each composition was substituted by a sodium hyaluronate raw material of the same molecular weight as a control.

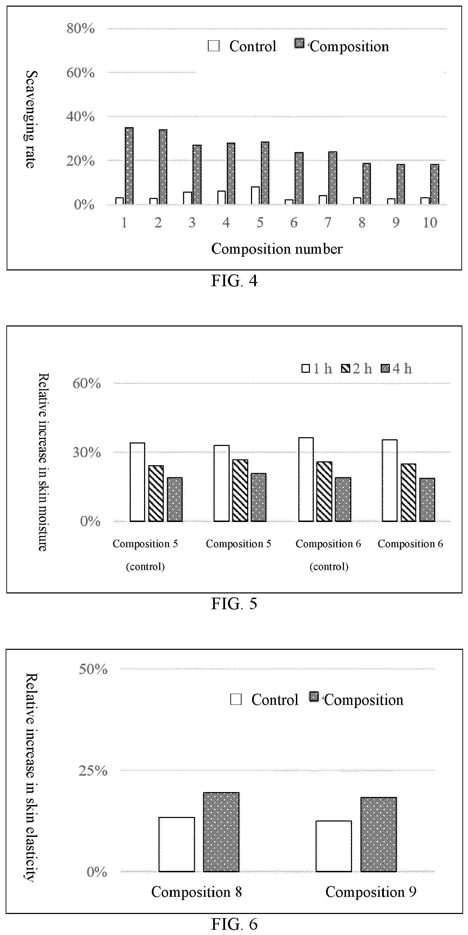

See FIG. 4 for the test results. The compositions of various proportions all had a significant anti-oxidation ability of scavenging free radicals, which was 3.5 to 12 times stronger than that of the control.

Embodiment 7: preparation of hyaluronic acid skin care compositions (including three or more molecular weights of sodium hyaluronate)

Thiolated hyaluronic acid derivatives used were prepared from 180 KDa sodium hyaluronate as a raw material by using the method reported by Shu et al. (Shu et al., Biomacromolecules 2002, 3:1304-1311), and their thiol contents were 18 $\mu$mol/g, 38 $\mu$mol/g, 57 $\mu$mol/g, 108 $\mu$mol/g, 266 $\mu$mol/g, 416 $\mu$mol/g, and 530 $\mu$mol/g respectively.

The sodium hyaluronate had molecular weights of 1.6 KDa, 3 KDa, 8 KDa, 50 KDa, 180 KDa, 500 KDa, 800 KDa, 1,500 KDa, and 2,700 KDa.

The thiolated hyaluronic acid derivative of any thiol content above was dissolved in distilled water to obtain a 6 mg/mL solution; the sodium hyaluronate (at any weight ratio) of any three or more molecular weights above was dissolved in distilled water to obtain a solution with a total concentration of 12 mg/mL; and then the two were mixed uniformly at a ratio of 0.9:0.1 to 0.1:0.9 to obtain hyaluronic acid skin care compositions of different proportions. In these compositions, the thiolated hyaluronic acid derivatives had any concentration of between 5.4 mg/mL and 0.6 mg/mL, and the sodium hyaluronate had any total concentration of between 1.2 mg/mL and 10.8 mg/mL.

In the compositions obtained, distilled water was added at a volume ratio of 1:0.08 to 0.08:1, and diluted compositions were obtained after uniform mixing. In the diluted compositions, the thiolated hyaluronic acid derivatives had any concentration of between 5.0 mg/mL and 0.04 mg/mL, and the total sodium hyaluronate had any concentration of between 0.09 mg/mL and 10.0 mg/mL.

The above compositions were filled in glass ampoules under the protection of an inert atmosphere (nitrogen) and sealed for later use.

Embodiment 8: preparation of hyaluronic acid skin care compositions (including two or more thiolated hyaluronic acid derivatives and three or more molecular weights of sodium hyaluronate)

Thiolated hyaluronic acid derivatives were prepared from 180 KDa, 300 KDa and 1,500 KDa sodium hyaluronate as raw materials by using the method reported by Shu et al. (Shu et al., Biomacromolecules 2002, 3:1304-1311), and their thiol contents were respectively 38 $\mu$mol/g, 33 $\mu$mol/g and 40 $\mu$mol/g.

The sodium hyaluronate had molecular weights of 1.6 KDa, 3 KDa, 8 KDa, 50 KDa, 180 KDa, 500 KDa, 800 KDa, 1,500 KDa, and 2,700 KDa.

Any two or more thiolated hyaluronic acid derivatives (any weight ratio) above were dissolved in distilled water to obtain a 6 mg/mL solution; the sodium hyaluronate (at any weight ratio) of any three or more molecular weights above was dissolved in distilled water to obtain a solution with a total concentration of 12 mg/mL; and then the two were mixed uniformly at a ratio of 0.9:0.1 to 0.1:0.9 to obtain hyaluronic acid skin care compositions of different proportions. In these compositions, the thiolated hyaluronic acid derivatives had any concentration of between 5.4 mg/mL and 0.6 mg/mL, and the sodium hyaluronate had any total concentration of between 1.2 mg/mL and 10.8 mg/mL.

In the compositions obtained, distilled water was added at a volume ratio of 1:0.08 to 0.08:1, and diluted compositions were obtained after uniform mixing. In the diluted compositions, the thiolated hyaluronic acid derivatives had any concentration of between 5.0 mg/mL and 0.04 mg/mL, and the total sodium hyaluronate had any concentration of between 0.09 mg/mL and 10.0 mg/mL.

The above compositions were filled in glass ampoules under the protection of an inert atmosphere (nitrogen) and sealed for later use.

Embodiment 9: moisturizing properties of hyaluronic acid skin care compositions

The hyaluronic acid skin care compositions in Embodiment 6 (composition numbers 5 and 6 and their respective controls) were selected, and 0.5 mL of each composition was uniformly applied to $3\times3$ cm$^2$ test areas on inner sides of left and right arms of healthy volunteers. Moisture contents of skin within the test areas were measured before application and 1, 2, and 4 hours after application (Cornrometer CM825).

The relative increase in skin moisture was calculated according to the following formula:

$$\text{Relative increase in skin moisture } (\%) = (\textbf{Fel!}) \times 100\%$$

See FIG. 5 for the test results. The composition of any proportion had good moisturizing ability, and the moisturizing property was close to that of the control, indicating that the thiolated hyaluronic acid derivative did not affect the moisturizing property of hyaluronic acid.

Embodiment 10: effects of hyaluronic acid skin care compositions on skin elasticity

The effects of hyaluronic acid compositions on skin elasticity were measured by a similar method disclosed in CN106137786B. Six subjects were recruited in each group. The hyaluronic acid skin care compositions (composition numbers 8 and 9 in Embodiment 6 and their respective controls) were applied to the cheek and gently massaged until all was absorbed. Each composition was applied once after face cleansing every morning and evening for 4 weeks. Elasticity changes of the cheek (apple cheek) were measured by a skin elasticity meter MPA580 (Courage + Khazaka, Germany) before application and 4 weeks after application.

Elasticity indexes of this test included initial elasticity, net elasticity and biological elasticity. The skin elasticity was represented by an average value of the three elasticity indexes. The relative increase in skin elasticity (%) was calculated according to the following formula:

$$\text{Relative increase in skin elasticity } (\%) = (\textbf{Fel!}) \times 100\%$$

See FIG. 6 for the test results. The composition of each proportion obviously increased skin elasticity, which was more significant than that of the control.

Embodiment 11: effects of hyaluronic acid skin care compositions on skin wrinkles

Effects of hyaluronic acid compositions on skin wrinkles were measured by a similar method disclosed in CN106137786B. Six subjects were recruited in each group. The hyaluronic acid skin care compositions (composition numbers 8 and 9 in Embodiment 6) were applied to canthi and gently massaged until all was absorbed. Each composition was applied once after face cleansing every morning and evening for 4 weeks. Depths of wrinkles on subjects' right eyes were respectively evaluated by using a fast three-dimensional imaging system for human skin before use and 4 weeks after use.

The relative decrease in average wrinkle depth (%) was calculated according to the following formula:

$$\text{Relative decrease in average wrinkle depth } (\%) = (\textbf{Fel!}) \times 100\%$$

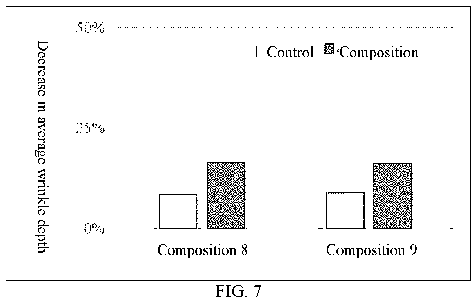

See FIG. 7 for the test results. The composition of each proportion significantly reduced depths of wrinkles, which was more significant than that of the control.

The above embodiments are preferred embodiments of the present disclosure, but the embodiments of the present disclosures are not limited by the foregoing embodiments. Any other changes, modifications, substitutions, combinations or simplifications made without departing from the spirit essence and principle of the present disclosure shall be equivalent replacements, and shall be included within the protection scope of the present disclosure.

**Claims**

1. A hyaluronic acid skin care composition, wherein the skin care composition comprises:

   1) a thiolated hyaluronic acid derivative; and
   2) a hyaluronic acid or salt of at least one molecular weight thereof.

2. The hyaluronic acid skin care composition according to claim 1, wherein the thiolated hyaluronic acid derivative has a thiol content of less than 500 $\mu$mol/g.

3. The hyaluronic acid skin care composition according to claim 2, wherein the thiolated hyaluronic acid derivative has a thiol content of less than 100 $\mu$mol/g.

4. The hyaluronic acid skin care composition according to claim 3, wherein the thiolated hyaluronic acid derivative has a thiol content of between 20 $\mu$mol/g and 50 $\mu$mol/g.

5. The hyaluronic acid skin care composition according to claim 1, wherein the thiolated hyaluronic acid derivative has a molecular weight of between 1 KDa and 10,000 KDa.

6. The hyaluronic acid skin care composition according to claim 5, wherein the thiolated hyaluronic acid derivative has a molecular weight of between 1.6 KDa and 3,000 KDa.

7. The hyaluronic acid skin care composition according to claim 6, wherein the thiolated hyaluronic acid derivative has a molecular weight of between 2.4 KDa and 1,500 KDa.

8. The hyaluronic acid skin care composition according to claim 1, wherein the thiolated hyaluronic acid derivative has a concentration of less than 5 mg/ml.

9. The hyaluronic acid skin care composition according to claim 8, wherein the thiolated hyaluronic acid derivative has a concentration of less than 2 mg/ml.

10. The hyaluronic acid skin care composition according to claim 9, wherein the thiolated hyaluronic acid derivative has a concentration of less than 1 mg/ml.

11. The hyaluronic acid skin care composition according to claim 1, wherein the hyaluronic acid or salt thereof is a sodium salt.

12. The hyaluronic acid skin care composition according to claim 1, wherein the hyaluronic acid or salt thereof has a molecular weight of between 1 KDa and 10,000 KDa.

13. The hyaluronic acid skin care composition according to claim 13, wherein the hyaluronic acid or salt thereof has a molecular weight of between 1.6 KDa and 3,000 KDa.

14. The hyaluronic acid skin care composition according to claim 13, wherein the hyaluronic acid or salt thereof has a molecular weight of between 2.4 KDa and 2,800 KDa.

15. The hyaluronic acid skin care composition according to claim 1, wherein the skin care composition comprises two or more molecular weights of hyaluronic acids or salts thereof at the same time.

16. The hyaluronic acid skin care composition according to claim 15, wherein at least one of the hyaluronic acids or salts thereof has a molecular weight of between 1.6 KDa and 500 KDa, and at least one of the hyaluronic acids or salts thereof has a molecular weight of between 800 KDa and 3,000 KDa.

17. The hyaluronic acid skin care composition according to claim 16, wherein at least one of the hyaluronic acids or salts thereof has a molecular weight of between 2.4 KDa and 8 KDa, and at least one of the hyaluronic acids or salts thereof has a molecular weight of between 1,000 KDa and 2,800 KDa.

18. The hyaluronic acid skin care composition according to claim 1, wherein the hyaluronic acid or salt thereof has a concentration of less than 10 mg/ml.

19. The hyaluronic acid skin care composition according to claim 18, wherein the hyaluronic acid or salt thereof has a concentration of less than 8 mg/ml.

20. The hyaluronic acid skin care composition according to claim 19, wherein the hyaluronic acid or salt thereof has a concentration of less than 4 mg/ml.

21. A preparation method of the hyaluronic acid skin protection composition according to any one of claims 1-20, wherein various components are mixed uniformly to obtain the composition.

22. The preparation method of the hyaluronic acid skin care composition according to claim 21, wherein the components are mixed uniformly and filled in a sealed container under the protection of an inert atmosphere.

23. An application of the hyaluronic acid skin care composition of any one of claims 1-20 in the preparation of skin cleansing, care and cosmetic products.

24. The application according to claim 23, wherein the skin cleansing, care and cosmetic products are selected from toners, essences, gels, lotions, creams, masks, makeup, soaps, facial cleansers, or shower gels.

25. The application according to claim 24, wherein the skin care composition has the effects of moisturizing, scavenging free radicals for anti-oxidation, restoring skin elasticity and reducing skin wrinkles in skin cleansing, care and cosmetics.

FIG. 1

FIG. 2

FIG. 3

FIG. 4

FIG. 5

FIG. 6

FIG. 7

## INTERNATIONAL SEARCH REPORT

| International application No. |
|---|
| **PCT/CN2019/127012** |

| A. | CLASSIFICATION OF SUBJECT MATTER |
|---|---|
| | A61K 8/73(2006.01)i; A61Q 19/08(2006.01)i; A61Q 19/00(2006.01)i |

According to International Patent Classification (IPC) or to both national classification and IPC

| B. | FIELDS SEARCHED |
|---|---|

Minimum documentation searched (classification system followed by classification symbols)

A61K, A61Q

Documentation searched other than minimum documentation to the extent that such documents are included in the fields searched

Electronic data base consulted during the international search (name of data base and, where practicable, search terms used)

WPI, EPODOC, CNKI, CNPAT, PubMed, ISI Web of Knowledge, STN, 常州百瑞吉, 王云云, 王昕宇, 宋文俊, 张红晨, 透明质酸, 透明质酸钠, 巯基化, 皮肤, 保湿, 抗氧化, DPPH, oxidation resistance, antioxidant, antioxidation, hyaluronic acid, skin, thiolated?, sulfydryl, hydrosulphonyl, hydrosulfuryl, thiol, antioxidative, cosmetic

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 110279610 A (BIOREGEN BIOMEDICAL (CHANGZHOU) CO., LTD.) 27 September 2019 (2019-09-27)<br>claims 1-25 | 1-25 |
| Y | CN 106137786 A (SHANDONG HUAXIHAIYU BIOLOGY MEDICINE CO., LTD.) 23 November 2016 (2016-11-23)<br>claims 1 and 8 | 1-25 |
| Y | CN 101622017 A (CROMA-PHARMA GESELLSCHAFT M.B.H) 06 January 2010 (2010-01-06)<br>claims 1-5, and description, page 2, the last paragraph | 1-25 |
| Y | 魏长征等 (WEI, Changzheng et al.). "巯基化透明质酸对退变性骨关节炎的潜在治疗研究 (Research on the Potential Treatment of Degenerative Osteoarthritis by Thiol-modified Hyaluronic Acid)"<br>中国骨与关节杂志 (Chinese Journal of Bone and Joint),<br>Vol. 4, No. 11, 30 November 2005 (2005-11-30),<br>pages 850-855 | 1-25 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **03 March 2020** | **26 March 2020** |

| Name and mailing address of the ISA/CN | Authorized officer |
|---|---|
| **China National Intellectual Property Administration (ISA/ CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

## INTERNATIONAL SEARCH REPORT

| | International application No. |
|---|---|
| | **PCT/CN2019/127012** |

**C. DOCUMENTS CONSIDERED TO BE RELEVANT**

| Category* | Citation of document, with indication, where appropriate, of the relevant passages | Relevant to claim No. |
|---|---|---|
| Y | CN 106109265 A (SHANDONG HUAXI HAIYU BIOLOGICAL MEDICINE CO., LTD.) 16 November 2016 (2016-11-16) <br> claims 1-8 | 1-25 |
| A | SHU, Xiaozheng et al. "Disulfide Cross-Linked Hyaluronan Hydrogels" <br> *Biomacromolecules,* Vol. 3, No. 6, 31 December 2002 (2002-12-31), <br> pp. 1304-1311 | 1-25 |

Form PCT/ISA/210 (second sheet) (January 2015)

**INTERNATIONAL SEARCH REPORT**
Information on patent family members

International application No.

**PCT/CN2019/127012**

| Patent document cited in search report | | | Publication date (day/month/year) | Patent family member(s) | | | Publication date (day/month/year) |
|---|---|---|---|---|---|---|---|
| CN | 110279610 | A | 27 September 2019 | None | | | |
| CN | 106137786 | A | 23 November 2016 | CN | 106137786 | B | 18 September 2018 |
| CN | 101622017 | A | 06 January 2010 | PL | 2107913 | T3 | 31 July 2012 |
| | | | | AT | 545436 | T | 15 March 2012 |
| | | | | WO | 2008077172 | A3 | 14 May 2009 |
| | | | | PT | 2107913 | E | 10 May 2012 |
| | | | | EP | 2107913 | A2 | 14 October 2009 |
| | | | | SI | EP2107913 | T1 | 31 May 2012 |
| | | | | CA | 2673323 | A1 | 03 July 2008 |
| | | | | AU | 2007336692 | A1 | 03 July 2008 |
| | | | | JP | 5513893 | B2 | 04 June 2014 |
| | | | | CY | 1112623 | T1 | 10 February 2016 |
| | | | | CN | 101622017 | B | 11 September 2018 |
| | | | | US | 2010028399 | A1 | 04 February 2010 |
| | | | | ES | 2391862 | T3 | 30 November 2012 |
| | | | | BR | PI0722061 | A2 | 01 April 2014 |
| | | | | RU | 2009128235 | A | 27 January 2011 |
| | | | | EP | 2107913 | B1 | 15 February 2012 |
| | | | | JP | 2010512859 | A | 30 April 2010 |
| | | | | SI | 2107913 | T1 | 31 May 2012 |
| | | | | DK | 2107913 | T3 | 21 May 2012 |
| | | | | CA | 2673323 | C | 16 July 2013 |
| | | | | BR | PI0722061 | B1 | 15 May 2018 |
| | | | | AU | 2007336692 | B2 | 12 December 2013 |
| | | | | US | 9597277 | B2 | 21 March 2017 |
| | | | | RU | 2432181 | C2 | 27 October 2011 |
| | | | | WO | 2008077172 | A2 | 03 July 2008 |
| CN | 106109265 | A | 16 November 2016 | CN | 106109265 | B | 21 August 2018 |

Form PCT/ISA/210 (patent family annex) (January 2015)

## REFERENCES CITED IN THE DESCRIPTION

**Patent documents cited in the description**

- CN 106109265 B
- CN 106137786 B
- CN 101450028 A
- CN 101500535 A
- WO 2009006780 A1
- US 7196180 B1, Aeschlimann
- US 6884788 B, Bulpitt
- WO 2005056608 A1, Prestwich
- CN 109206537 A
- US 20130338099 A1, Shu
- CN 101622017 B

**Non-patent literature cited in the description**

- **GUTTERIDGE et al.** *British Medical Bulletin,* 1999, vol. 55, 49-57
- **AGREN et al.** *Free Radical Biol. Med.,* 1997, vol. 23, 996-1001
- **STERN et al.** *Clinics in Dermatology,* 2008, vol. 26, 106-122
- **SMEJKALOVA et al.** Harry's Cosmeticology. Chemical Publishing Company, 2015, vol. 2, 605-622
- Biomatrix Inc. Hyaluronan. Biomedical, Medical and Clinical Aspects. Woodhead Publishing Limited, 2002, vol. 2, 285-288
- **FARWICK et al.** *SOFW-Journal,* 2008, vol. 134, 1-6
- **YAMAZAKI et al.** *Pathophysiology,* 2003, vol. 9, 215-220
- **STERN et al.** *Biotechnology Advances,* 2007, vol. 25, 537-557
- **SERBAN et al.** *Biomaterials,* 2008, vol. 29, 1388-1399
- **SHU et al.** *Biomacromolecules,* 2002, vol. 3, 1304-1311
- **GIANOLIO et al.** *Bioconjugate Chemistry,* 2005, vol. 16, 1512-1518
- **KAFEDJIISKI et al.** *International Journal of Pharmaceutics,* 2007, vol. 343, 48-58
- **INOUE et al.** *Carbohydrate Research,* 1985, vol. 141, 99-110
- **SHARMA et al.** *Food Chemistry,* 2009, vol. 113, 1202-1205